(19) Europäisches Patentamt
European Patent Office
Office européen des brevets

(11) **EP 2 549 264 A1**

(12) **EUROPÄISCHE PATENTANMELDUNG**

(43) Veröffentlichungstag:
**23.01.2013 Patentblatt 2013/04**

(51) Int Cl.:
***G01N 21/27*** *(2006.01)*

(21) Anmeldenummer: **11174320.9**

(22) Anmeldetag: **18.07.2011**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(71) Anmelder: **Siemens Healthcare Diagnostics Products GmbH
35041 Marburg (DE)**

(72) Erfinder:
• **Sass, Karl**
**35274 Kirchhain (DE)**
• **Greis, Dirk**
**35099 Burgwald (DE)**
• **Noah, Michael Dr.**
**35041 Marburg (DE)**
• **Ueckermann, Christian**
**35041 Marburg (DE)**

(54) **Verfahren und System zum Bestimmen der Konzentration von Substanzen in Körperflüssigkeiten**

(57) Die Erfindung betrifft ein Verfahren zur spektrophotometrischen Bestimmung der Konzentrationen von mehreren Substanzen, bevorzugt von Bilirubin, Hämoglobin und Lipiden in einer Körperflüssigkeitsprobe.

# FIG 2

EP 2 549 264 A1

**Beschreibung**

Die vorliegende Erfindung bezieht sich auf ein Verfahren und ein System zum Bestimmen der Konzentration von Substanzen in Körperflüssigkeiten, insbesondere von Störsubstanzen wie Bilirubin, Hämoglobin und Lipiden in Blutserum- und Blutplasmaproben.

Zahlreiche Nachweis- und Analyseverfahren zur Bestimmung physiologischer Parameter in Körperflüssigkeitsproben beruhen auf photometrischen Messprinzipien. Photometrische Verfahren ermöglichen den qualitativen und quantitativen Nachweis von Analyten in flüssigen Proben.

Die Bestimmung klinisch relevanter Parameter, wie zum Beispiel der Konzentration oder der Aktivität eines Analyten erfolgt vielfach, indem ein Aliquot einer Körperflüssigkeit eines Patienten mit einem oder mehreren Testreagenzien in vitro vermischt wird, wodurch eine biochemische Reaktion in Gang gesetzt wird, die eine messbare Veränderung einer optischen Eigenschaft des Testansatzes bewirkt. Die Photometrie untersucht und nutzt die Schwächung eines Lichtstroms beim Durchtritt durch ein absorbierendes und/oder streuendes Medium. Je nach Art der ausgelösten biochemischen oder biophysikalischen Reaktion kommen unterschiedliche photometrische Messverfahren zum Einsatz, die die Messung eines trüben flüssigen Testansatzes ermöglichen.

Hierzu können turbidimetrische Verfahren eingesetzt werden, bei denen die Trübung beziehungsweise die optische Dichte einer Lösung oder Suspension anhand der Lichtschwächung oder Extinktion eines direkt durch die Suspension hindurch tretenden Lichtstrahls gemessen wird.

Die Intensität des Lichtstrahls nimmt beim Durchtritt durch eine Messzelle beziehungsweise Küvette, die eine flüssige Probe enthält, ab. Die Verluste können durch Interaktionen des Lichtstrahls mit der in der Messzelle befindlichen Probe, beispielsweise durch Absorptions-, Diffraktions-, Streuungs-und/oder Reflexionseffekte beeinflusst werden. Im Allgemeinen können Diffraktions-, Beugungs- und Reflexionseffekte vernachlässigt beziehungsweise durch Referenzmessungen ausgeglichen werden, so dass hauptsächlich die Absorption zur Schwächung des Lichtstrahls beiträgt.

Photometrische Konzentrationsbestimmungen beruhen daher auf einer gesetzmäßigen Abhängigkeit der Extinktion beziehungsweise Absorption von der Konzentration der gelösten Stoffe und der Schichtdicke der Messzelle bei einer bestimmten Wellenlänge des eingestrahlten Lichts. Diesen Zusammenhang beschreibt das Lambert-Beersche Gesetz:

$$E(\lambda) = -\log(I/I_0) = \varepsilon(\lambda) \cdot c \cdot d \qquad (1)$$

wobei $E(\lambda)$ die von der Wellenlänge $\lambda$ des Lichtstrahls abhängige Extinktion, $I$ die Lichtintensität nach Durchtritt durch die Probe, $I_0$ die Lichtintensität vor Durchtritt durch die Probe, $\varepsilon(\lambda)$ der wellenlängenabhängige molare Extinktionskoeffizient eines durchstrahlten Stoffes, $c$ die molare Konzentration des durchstrahlten Stoffes und $d$ die durch den Lichtstrahl durchstrahlte Schichtdicke, beispielsweise der Messzelle ist.

Anhand der Extinktion $E(\lambda)$ einer Probe lässt sich die Konzentration einer Substanz in einer Lösung ermitteln. Dazu ist es erforderlich, dass zuvor die Extinktion mindestens einer Standardlösung bekannter Konzentration bestimmt wurde. Da sich die Extinktion proportional zur Konzentration verhält, kann mittels Kalibration durch Extinktionsmessungen mehrerer Standardlösungen bekannter Konzentrationen die Konzentration einer gelösten Substanz ermittelt werden.

Die Extinktion einer Probe hängt jedoch nicht nur von der Konzentration der zu bestimmenden Substanz selbst ab, sondern auch von der Art der Probenmatrix. Die Extinktionen verschiedener Substanzen verhalten sich in einem Gemisch additiv, sofern die Substanzen nicht untereinander wechselwirken. Körperflüssigkeiten, wie beispielsweise Blutplasma oder Blutserum sind jeweils komplexe Gemische und enthalten neben dem zu bestimmenden Analyten eine Vielzahl weiterer Substanzen, die die Gesamtabsorption der Probe beeinflussen.

Körperflüssigkeitsproben können jedoch in Einzelfällen abnormal hohe Konzentrationen einer oder mehrerer intrinsischer, also körpereigener Substanzen enthalten, die sich bei Überschreitung einer tolerablen Konzentration in photometrischen Detektionsverfahren als störend erweisen und sich zu einem systematischen Fehler auswirken können.

Probleme bereiten bekanntermaßen hämolytische, ikterische und/oder lipämische Serum- oder Plasmaproben, die über abnormal hohe Hämoglobin-, Bilirubin- und/oder Lipid-Konzentrationen verfügen. Abnormal hohe Konzentrationen dieser interferierenden Substanzen können durch einen pathologischen Zustand des Patienten oder aber durch eine unsachgemäße Probengewinnung oder -lagerung verursacht werden. Werden solche Proben einem photometrischen Verfahren unterworfen, das der Bestimmung eines analytischen, diagnostisch relevanten Parameters dient, besteht die Gefahr einer Fehlbestimmung die gegebenenfalls eine Fehldiagnose und schlimmstenfalls eine Fehlbehandlung des Patienten zur Folge haben kann. Die präanalytische Identifikation hämolytischer, ikterischer sowie lipämischer Proben ist also zur Vermeidung von fehlerhaften Analyseergebnissen von besonderer Wichtigkeit.

Es besteht daher ein Bedarf an Verfahren zur Ermittlung der spektrometrischen Auswirkungen störender Substanzen in Körperflüssigkeitsproben.

In EP-A1-1059522, US 4,263,512, US 2009/0009750 A1 und US 2010/0174491 A1 sind verschiedene Verfahren zur Bestimmung von Bilirubin, Hämoglobin und Lipiden in Plasma- oder Serumproben beschrieben.

Die bekannten Verfahren haben jedoch den Nachteil, dass eine hohe Lipidkonzentration die Bestimmung von Bilirubin und Hämoglobin in derselben Probe beeinflussen kann.

Der vorliegenden Erfindung liegt demnach die Aufgabe zugrunde, ein Verfahren zur spektrophotometrischen Bestimmung mehrerer Substanzen in einer Körperflüssigkeitsprobe bereit zu stellen, das auch in Körperflüssigkeitsproben mit hohen Lipidkonzentrationen eine zuverlässige Bestimmung anderer Substanzen, wie beispielsweise Hämoglobin und Bilirubin, ermöglicht.

Diese Aufgabe wird durch das erfindungsgemäße Verfahren gelöst.

Eine Ausführungsform der vorliegenden Erfindung besteht in einem Verfahren zum Bestimmen der Konzentrationen von Substanzen in einer Körperflüssigkeitsprobe, mit den Schritten des Durchstrahlens einer eine erste und eine zweite und gegebenenfalls eine dritte Substanz enthaltenden Körperflüssigkeitsprobe mit einem Lichtstrahl bei einer Vielzahl von Lichtwellenlängen und des Erfassens einer Vielzahl von Messwerten der Extinktion der Körperflüssigkeitsprobe bei der Vielzahl von Wellenlängen, des Berechnens einer Näherungskurve für die Extinktion einer ersten Substanz auf der Basis eines ersten Messwerts bei einer ersten Wellenlänge, des Bestimmens eines ersten Näherungswerts der Konzentration der zweiten Substanz auf der Basis eines zweiten Messwerts und Werten der Näherungskurve bei einer zweiten Wellenlänge, des Berechnens eines Extinktionswerts bei einer dritten Wellenlänge auf der Basis des ersten Näherungswerts und Werten der Näherungskurve, des Ermittelns einer Abweichung des berechneten Extinktionswerts von einem dritten Messwert bei der dritten Wellenlänge, des Korrigierens der Näherungskurve auf der Basis der ermittelten Abweichung, und des Korrigierens des ersten Näherungswerts auf der Basis des zweiten Messwerts und Werten der korrigierten Näherungskurve.

Gemäß einer vorteilhaften Ausführungsform kann die Körperflüssigkeitsprobe weiterhin eine dritte Substanz enthalten, und es können weiterhin die Schritte des Bestimmens eines zweiten Näherungswerts der Konzentration einer dritten Substanz auf der Basis des zweiten Messwerts und Werten der Näherungskurve bei einer zweiten Wellenlänge sowie eines vierten Messwerts und Werten der Näherungskurve bei einer vierten Wellenlänge, und des Korrigierens des zweiten Näherungswerts auf der Basis des zweiten Messwerts, des vierten Messwerts und Werten der korrigierten Näherungskurve, wobei der Extinktionswert zusätzlich auf der Basis des zweiten Näherungswertes berechnet wird.

In einer bevorzugten Ausführungsform können die Schritte des Berechnens des Extinktionswerts, des Ermittelns der Abweichung und des Korrigierens der Näherungskurve, des ersten Näherungswerts und des zweiten Näherungswerts so lange iteriert werden, bis die Abweichung unterhalb eines vorbestimmten Schwellwerts liegt.

Vorteilhafterweise kann die Körperflüssigkeitsprobe Blutserum oder Blutplasma und die erste Substanz Lipide umfassen. Weiterhin ist es vorteilhaft, aber nicht notwendig, wenn die zweite Substanz Hämoglobin und die dritte Substanz Bilirubin umfasst.

Gemäß einer bevorzugten Ausführungsform liegt die erste Wellenlänge im Bereich zwischen 610 nm und 650 nm, die zweite Wellenlänge im Bereich zwischen 410 nm und 420 nm, die dritte Wellenlänge im Bereich zwischen 360 nm und 370 nm und die vierte Wellenlänge im Bereich zwischen 465 nm und 475 nm. Gemäß einer vorteilhaften Ausführungsform beträgt der vorbestimmte Schwellwert 0,01 E.

Vorteilhafterweise kann das Korrigieren der Näherungskurve derart erfolgen, dass der erste Messwert auf der Näherungskurve liegt.

Gemäß einer vorteilhaften Ausführungsform kann das Durchstrahlen der Körperflüssigkeitsprobe mithilfe von Laser- oder Leuchtdioden und das Erfassen der Vielzahl von Messwerten mithilfe eines photometrischen Sensors erfolgen.

Die vorliegende Erfindung schafft in einer weiteren Ausführungsform ein System, z.B. ein Analysegerät, zum Bestimmen der Konzentrationen von Substanzen in einer Körperflüssigkeitsprobe, mit einer Messeinrichtung, welche dazu ausgelegt ist, eine erste und/oder zweite Substanzen enthaltende Körperflüssigkeitsprobe mit einem Lichtstrahl bei einer Vielzahl von Lichtwellenlängen zu durchstrahlen, und eine Vielzahl von Messwerten der Extinktion der Körperflüssigkeitsprobe bei der Vielzahl von Wellenlängen zu erfassen, und einer Berechnungseinrichtung, welche dazu ausgelegt ist, eine Näherungskurve für die Extinktion einer ersten Substanz auf der Basis eines ersten Messwerts bei einer ersten Wellenlänge zu berechnen, einen ersten Näherungswert der Konzentration der zweiten Substanz auf der Basis eines zweiten Messwerts und Werten der Näherungskurve bei einer zweiten Wellenlänge zu bestimmen, einen Extinktionswert bei einer dritten Wellenlänge auf der Basis des ersten Näherungswerts und Werten der Näherungskurve zu berechnen, eine Abweichung des berechneten Extinktionswerts von einem dritten Messwert bei der dritten Wellenlänge zu ermitteln, die Näherungskurve auf der Basis der ermittelten Abweichung zu korrigieren und den ersten Näherungswert auf der Basis des zweiten Messwerts und Werten der korrigierten Näherungskurve zu korrigieren.

Vorteilhafterweise kann die Messeinrichtung Laser- oder Leuchtdioden und eine photometrische Sensoreinrichtung aufweisen.

In einer bevorzugten Ausführungsform kann die Berechnungseinrichtung weiterhin dazu ausgelegt sein, einen zweiten Näherungswert der Konzentration einer dritten Substanz auf der Basis des zweiten Messwerts und Werten der Näherungskurve bei einer zweiten Wellenlänge sowie eines vierten Messwerts und Werten der Näherungskurve bei einer vierten Wellenlänge zu bestimmen, und den zweiten Näherungswert auf der Basis des zweiten Messwerts und Werten der korrigierten Näherungskurve zu korrigieren, wobei der Extinktionswert zusätzlich auf der Basis des zweiten Näherungswertes berechnet wird.

Weitere Modifikationen und Variationen ergeben sich aus den Merkmalen der abhängigen Ansprüche.

Kurze Beschreibung der Figuren

Verschiedene Ausführungsformen und Ausgestaltungen der vorliegenden Erfindung werden nun in Bezug auf die beiliegenden Zeichnungen genauer beschrieben.

Fig. 1     zeigt eine schematische Darstellung eines Diagramms mit Extinktionskurven von Lipiden gemäß einer Ausführungsform der Erfindung;

Fig. 2     zeigt eine schematische Darstellung eines Diagramms mit Extinktionskurven von Körperflüssigkeitsproben gemäß einer weiteren Ausführungsform der Erfindung;

Fig. 3     zeigt eine schematische Darstellung eines Verfahrens zum Bestimmen der Konzentrationen von Substanzen in einer Körperflüssigkeitsprobe gemäß einer weiteren Ausführungsform der Erfindung;

Fig. 4     zeigt eine schematische Darstellung eines Diagramms mit Extinktionskurven von Körperflüssigkeitsproben gemäß einer weiteren Ausführungsform der Erfindung;

Fig. 5     zeigt eine schematische Darstellung eines Diagramms mit Extinktionskurven von Körperflüssigkeitsproben gemäß einer weiteren Ausführungsform der Erfindung; und

Fig. 6     zeigt eine schematische Darstellung eines Systems zum Bestimmen der Konzentrationen von Substanzen in einer Körperflüssigkeitsprobe gemäß einer weiteren Ausführungsform der Erfindung zeigt.

Die beschriebenen Ausgestaltungen und Weiterbildungen lassen sich, sofern sinnvoll, beliebig miteinander kombinieren. Weitere mögliche Ausgestaltungen, Weiterbildungen und Implementierungen der Erfindung umfassen auch nicht explizit genannte Kombinationen von zuvor oder im Folgenden bezüglich der Ausführungsbeispiele beschriebenen Merkmale der Erfindung.

Die beiliegenden Zeichnungen sollen ein weiteres Verständnis der Ausführungsformen der Erfindung vermitteln. Sie veranschaulichen Ausführungsformen und dienen im Zusammenhang mit der Beschreibung der Erklärung von Prinzipien und Konzepten der Erfindung. Andere Ausführungsformen und viele der genannten Vorteile ergeben sich im Hinblick auf die Zeichnungen. Die Elemente der Zeichnungen sind nicht notwendigerweise maßstabsgetreu zueinander gezeigt. Gleiche Bezugszeichen bezeichnen dabei gleiche oder ähnlich wirkende Komponenten.

Körperflüssigkeitsproben im Sinne der vorliegenden Erfindung können alle Proben biologischen Ursprungs sein, welche flüssige Konsistenz aufweisen und eine Vielzahl von biologisch aktiven Substanzen in verschiedenen Konzentrationen aufweisen. Beispielsweise können Körperflüssigkeitsproben Blutserum, Blutplasma, Blut, Urin, Lymphflüssigkeit, Gallenflüssigkeit oder ähnliche Flüssigkeiten aufweisen.

Photometrische Messwerte im Sinne der vorliegenden Erfindung können Messwerte sein, welche mit photometrischen Messeinrichtungen und zugehörigen Lichtquellen, insbesondere Lasern, Laserdioden, Leuchtdioden oder dergleichen aufgenommen werden können. Messeinrichtungen umfassen beispielsweise CCD-Sensoren, CMOS-Sensoren, Photosensoren oder ähnliche Einrichtungen, welche dazu geeignet sind, die Intensität eines Lichtstrahls wellenlängenabhängig zu erfassen.

Lipide im Sinne der vorliegenden Anmeldung können alle im Wesentlichen hydrophoben organischen Verbindungen umfassen, insbesondere im menschlichen oder tierischen Organismus vorkommende Verbindungen. Lipide im Sinne der Erfindung umfassen dabei insbesondere Fette bzw. Triglyceride bzw. Triacylglycerine, welche im menschlichen Körper vorkommen können.

Extinktionskurven und Extinktionswerte im Sinne der vorliegenden Erfindung können dimensionslose Größen sein, welche ein wellenlängenabhängiges Maß für die Opazität von Körperflüssigkeitsproben gegenüber dem Durchgang von Lichtstrahlen im sichtbaren, infraroten und/oder ultravioletten Wellenlängenbereich angeben. Es kann gleichermaßen auch möglich sein, dass Extinktionswerte im Bezug auf eine Einheitsdicke einer Messzelle oder Küvette, in der Körperflüssigkeitsproben während des Durchtritts von Lichtstrahlen zur Erfassung von Intensitätsmesswerten gehalten werden, angegeben werden. In diesem Fall können die Extinktionswerte eine Dimension von [1/cm] aufweisen. In jedem Fall sind die angegebenen Extinktionswerte der nachfolgenden Ausführungsformen nur beispielhafter Natur und von der Messapparatur, der Probenbeschaffenheit und der Probenzusammensetzung abhängig. Extinktionswerte werden im Folgenden jeweils mit Absorptionswerten gleichgesetzt, obwohl es dem Fachmann klar ist, dass bei dieser Betrachtung Diffraktion, Streuung und Reflexion zwar zu den Extinktionswerten beitragen, gegenüber der Absorption jedoch im betrachteten Wellenlängenbereich im Wesentlichen vernachlässigbar sind.

In Körperflüssigkeitsproben können häufig Hämoglobin, Bilirubin und Lipide, insbesondere Triacylglycerine (Triglyceride), enthalten sein. Zur Bestimmung der Hämoglobin- und Bilirubinkonzentrationen mittels photospektrometrischer Untersuchungsmethoden ist es wichtig, den Lipidanteil zu bestimmen.

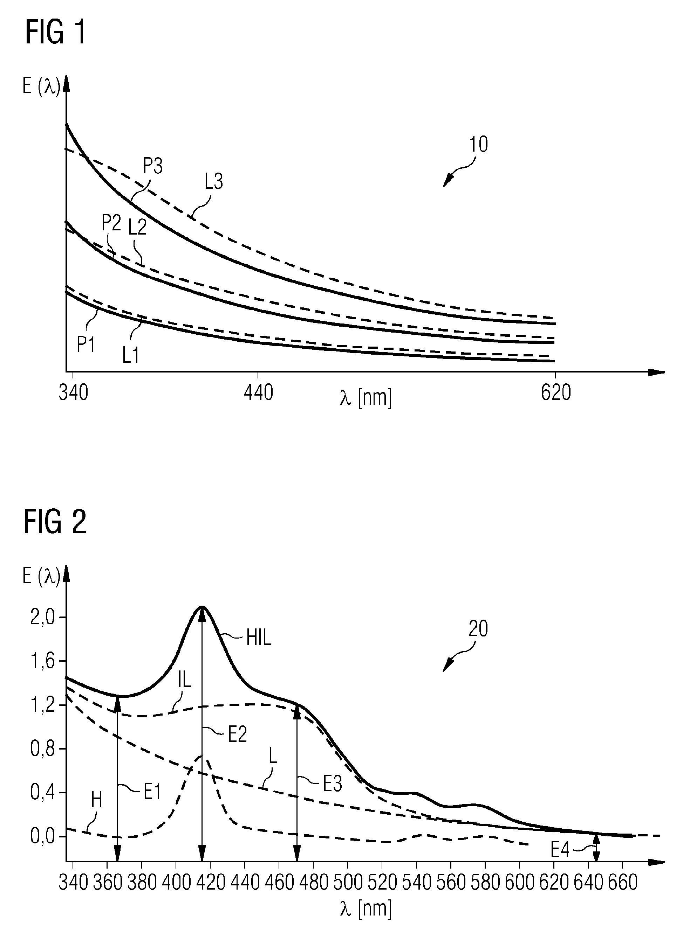

Fig. 1 zeigt eine schematische Darstellung eines Diagramms mit Extinktionskurven von Lipiden in einem Wellenlängenbereich zwischen etwa 340 nm und 620 nm. Die Extinktionskurven L1, L2 und L3 bilden jeweils künstlich angesetzte Lipid-Emulsionen (Intralipid, Lipovenös®) in den Lipid-Konzentrationen 60 mg/dl, 180 mg/dl bzw. 300 mg /dl. Zunächst lässt sich erkennen, dass die Extinktionswerte insgesamt mit der Lipidkonzentration ansteigen. Weiterhin lässt sich erkennen, dass im roten sichtbaren Spektralbereich um 600 nm bis 620 nm die Extinktion für alle Lipid-Konzentrationen geringer ist als die Extinktion im blauen sichtbaren bzw. ultravioletten Spektralbereich. Die Lipidkurven L1, L2 und L3 sind in Fig. 1 jeweils durch eine Potenzfunktion

$$E(\lambda) = p \cdot \lambda^{-q} \qquad\qquad (2)$$

angenähert worden, so dass sich die Näherungskurven P1, P2 und P3 mit jeweils angepassten Näherungsparametern p und q ergeben. Die Näherungskurven können beispielsweise mithilfe von Regressionsanalyseverfahren, beispielsweise von Kleinster-Quadrate-Schätzung, Maximum-Likelihood-Verfahren oder anderer Näherungsmethoden bestimmt werden.

Für die Lipidkurve L1 der niedrigsten Konzentration stimmt die Potenzkurve P1 gut mit dem tatsächlichen Extinktionsverlauf überein. Für höhere Konzentrationen jedoch können die Näherungskurven P2 und P3 die jeweiligen Extinktionsverläufe L2 bzw. L3 nicht mehr adäquat abbilden, insbesondere nicht im blauen Wellenlängenbereich zwischen etwa 340 nm und 470 nm. Die Extinktionsverläufe L2 und L3 bilden in diesem Bereich ein gewisses Extinktionsplateau aus, was durch Mehrfachstreuung verursacht wird. Insgesamt lässt sich feststellen, dass bei höheren Konzentrationen Extinktionskurven von Lipid-Emulsionen nicht ausreichend genau abgebildet werden können.

Fig. 2 zeigt eine schematische Darstellung eines Diagramms mit Extinktionskurven von Körperflüssigkeitsproben, insbesondere von Blutserum oder Blutplasma, im Wellenlängenbereich zwischen 340 nm und 660 nm. Blutserum oder Blutplasma können als emulgierte Substanzen Hämoglobin (H), Bilirubin (I) und Lipide (L) umfassen. Eine Bestimmung der Konzentration dieser Substanzen in der Körperflüssigkeitsprobe wird daher häufig auch als HIL-Check bezeichnet.

Die Extinktionskurve HIL gibt einen beispielhaften schematischen Verlauf für die wellenlängenabhängige Extinktion von Körperflüssigkeitsproben mit typischen Konzentrationen von Hämoglobin, Bililrubin und Lipide wider. Die Extinktionskurve kann dabei in einen Hämoglobinanteil H und einen kombinierten Lipid-/Bilirubinanteil IL aufgeteilt werden, deren geschätzte Extinktionskurven als gestrichelte Kurven in Fig. 2 dargestellt sind. Der reine Lipidanteil L ist ebenfalls als getrichelte Kurve dargestellt.

In einem roten Wellenlängenbereich zwischen etwa 610 nm und 650 nm ist die durch Hämoglobin und Bilirubin verursachte Extinktion vernachlässigbar. Daher kann mit einer ersten Messung bei einer ersten Wellenlänge zwischen 610 nm und 650 nm, beispielsweise bei 620 nm oder 645 nm ein erster Messwert E4 ermittelt werden, mit welchem über die Abhängigkeit des Lambert-Beerschen Gesetzes die molare Lipidkonzentration $C_L$ [L/ (mol*cm) ] in einer ersten Näherung bestimmt werden kann:

$$c_L = E4 / \varepsilon_{L4} \, , \qquad\qquad (3)$$

wobei $\varepsilon_{L4}$ der molare Extinktionskoeffizient von Triacylglycerinen bei der ersten Wellenlänge ist.

Vorzugsweise kann die Konzentration auch mit Hilfe eines gewichtsspezifischen Extinktionskoeffizienten bestimmt werden. Dazu wird in der Lambert-Beerschen Formel (Formel 1) die Weglänge d mit 1 mm gleichgesetzt, und aus dem Produkt des molaren Extinktionskoeffizienten $\varepsilon_{mol}$ und der Schichtdicke der Messzelle d wird der gewichtsspezifische Extinktionskoeffizient $\varepsilon$ bestimmt. Dies ermöglicht eine Bestimmung der Konzentration der Substanz in [mg/dL].

In weiteren Messungen können Messwerte E2 und E3 erfasst werden, welche in Wellenlängenbereichen liegen, in denen Extinktionsmaxima von Hämoglobin beziehungsweise Bilirubin liegen. Beispielsweise kann der Messwert E2 in einem Wellenlängenbereich zwischen 410 nm und 420 nm, insbesondere bei etwa 415 nm erfasst werden. Der Messwert E3 kann beispielsweise in einem Wellenlängenbereich zwischen 465 nm und 475 nm, insbesondere bei etwa 470 nm erfasst werden. Der Messwert E2 setzt sich aus Extinktionsanteilen zusammen, die Hämoglobin ($E_{H2}$), Bilirubin ($E_{I2}$) und Lipiden ($E_{L2}$) zugeschrieben werden können:

$$E2 = E_{H2} + E_{I2} + E_{L2} \; . \tag{4}$$

Gleichermaßen setzt sich der Messwert E3 aus Extinktionsanteilen zusammen, die Hämoglobin ($E_{H3}$), Bilirubin ($E_{I3}$) und Lipiden ($E_{L3}$) zugeschrieben werden können:

$$E3 = E_{H3} + E_{I3} + E_{L3} \; . \tag{5}$$

Schließlich kann ein Messwert E1 in einem Wellenlängenbereich erfasst werden, der als Kontrollbereich dienen kann, beispielsweise in einem Bereich zwischen 360 nm und 370 nm, insbesondere bei etwa 365 nm. Der Messwert E1 setzt sich aus Extinktionsanteilen zusammen, die Hämoglobin ($E_{H1}$), Bilirubin ($E_{I1}$) und Lipiden ($E_{L1}$) zugeschrieben werden können:

$$E1 = E_{H1} + E_{I1} + E_{L1} \; . \tag{6}$$

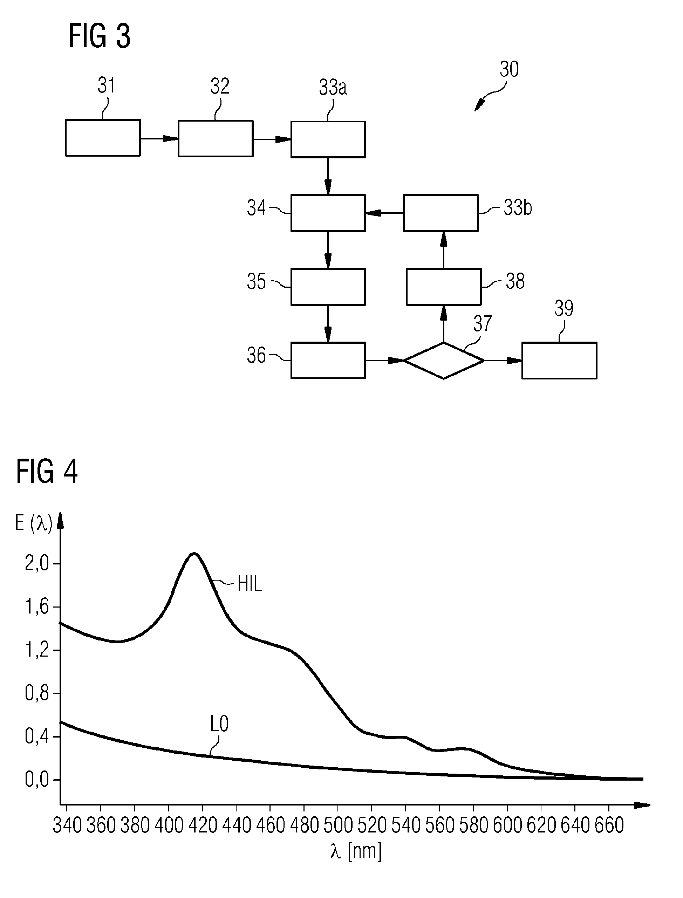

Fig. 3 zeigt eine schematische Darstellung eines Verfahrens 30 zum Bestimmen der Konzentrationen von Substanzen in einer Körperflüssigkeitsprobe, insbesondere von Hämoglobin, Bilirubin und Lipiden in einer Blutserum- oder Blutplasmaprobe.

In einem ersten Schritt 31 können Messwerte E1, E2, E3 und E4, wie im Zusammenhang mit Fig. 2 erläutert, erfasst werden. In einem zweiten Schritt 32 können erste Näherungsparameter $p_0$ und $q_0$ für eine erste Näherungskurve $L_0$ mithilfe einer Regressionsanalyse bestimmt werden. Dabei kann die Näherungskurve $L_0$ eine Potenzfunktion abbilden, die mit einem vorbestimmten Exponenten $q_0$ gebildet wird. Gemäß Gleichung (2) kann dann der Koeffizient $p_0$ über den Messwert E4 bei der ersten Wellenlänge bestimmt werden. Die Näherungskurve kann eine erste Näherung für den Extinktionsverlauf der Extinktion von Lipiden in der Probe wiedergeben. Hierzu kann für alle Wellenlängen, in denen in Schritt 31 weitere Messwerte erfasst worden sind, in Schritt 33a der jeweilige Extinktionsanteil $E_{L1}$, $E_{L2}$, $E_{L3}$ und $E_{L4}$ (= E4), der Lipiden berechnet werden.

Wie sich in Fig. 4 erkennen lässt, ergibt sich dadurch eine erste Näherungskurve $L_0$, welche in einem blauen bzw. ultravioletten Spektralbereich flacher verläuft als der tatsächliche Extinktionsverlauf für Lipide.

In den Schritten 34 und 35 können dann erste Näherungswerte für die Konzentrationen von Hämoglobin ($C_H$) und Bilirubin ($c_I$) auf der Basis der Messwerte E2 und E3, beispielsweise bei den Wellenlängen 415 nm und 470 nm, bestimmt werden:

$$c_I = \frac{E3 - c_H \cdot \varepsilon_{H3} - E_{L3}}{\varepsilon_{I3}} \tag{7}$$

$$c_H = \frac{E2 - c_I \cdot \varepsilon_{I2} - E_{L2}}{\varepsilon_{H2}} \tag{8}$$

wobei $\varepsilon_{H2}$, $\varepsilon_{H3}$, $\varepsilon_{I2}$ und $\varepsilon_{I3}$ die jeweiligen Extinktionskoeffizienten von Hämoglobin (H) und Bilirubin (I) bei den Wellenlängen der Messwerte E2 und E3 sind. Die Extinktionskoeffizienten können dabei vorab durch Referenzmessungen bestimmt werden, oder aus einem Speicher, in dem Referenzwerte abgelegt sind, für die Berechnungen abgerufen werden.

Zur Ermittlung der beiden Konzentrationen $C_I$ und $C_H$ kann das lineare Gleichungssystem der beiden Gleichungen (7) und (8) gelöst werden, so dass sich für die Konzentration von Hämoglobin (H) die Formel

$$c_H = \frac{E3 - E_{L3} - \frac{(E2 - E_{L2}) \cdot \varepsilon_{I3}}{\varepsilon_{I2}}}{\varepsilon_{H3} - \frac{\varepsilon_{H2} \cdot \varepsilon_{I3}}{\varepsilon_{I2}}} \qquad (9)$$

ergibt. Der erste Näherungswert für die Konzentration $C_H$ kann dann zur Bestimmung des ersten Näherungswerts für die Konzentration $C_I$ in Gleichung (7) eingesetzt werden.

In einem Schritt 36 kann dann ein Extinktionswert $E_{HIL}$ ermittelt werden, welcher einem Näherungswert für die gesamte Extinktion bei einer Wellenlänge zwischen 360 nm und 370 nm, beispielsweise 365 nm, entspricht:

$$E_{HIL} = c_H \cdot \varepsilon_{H1} + c_I \cdot \varepsilon_{I1} + E_{L1} \qquad (10)$$

In einem Schritt 37 kann dann ein Vergleich zwischen dem Wert $E_{HIL}$ und dem tatsächlichen Messwert E1 bei dieser Wellenlänge durchgeführt werden, um eine Abweichung

$$\Delta E = E1 - E_{HIL} \qquad (11)$$

zu erhalten. Wenn die Abweichung $\Delta E$ größer als ein vorbestimmter Schwellwert ist, kann bestimmt werden, dass die ermittelte Näherungskurve $L_0$ für die Konzentrationen der Substanzen nicht hinreichend genau genug ermittelt worden ist. In diesem Fall kann in einem Schritt 38 ein Korrigieren der Näherungskurve $L_0$ erfolgen. Hierzu kann der berechnete Extinktionswert $E_{L1}$, welcher den Extinktionsanteil von Lipid bei der Wellenlänge 365 nm beschreibt, um einen prozentualen Anteil der Abweichung $\Delta E$ korrigiert werden. Beispielsweise kann zu dem Extinktionswert $E_{L1}$ die Hälfte des Wertes der Abweichung $\Delta E$ addiert werden. Auf der Basis des korrigierten Extinktionswertes $E_{L1}$ kann dann eine korrigierte Näherungskurve $L_k$ mit den Parametern $p_k$ und $q_k$ bestimmt werden

$$q_k = \frac{\ln E4 - \ln(E_{L1} + \Delta E/2)}{\ln \lambda(E4) - \ln \lambda(E1)} \qquad (12)$$

$$p_k = \frac{E4}{\lambda(E4)^{-q_k}} \qquad (13)$$

Dabei kann die Näherungskurve $L_0$ derart korrigiert werden, dass der Messwert E4, beispielsweise bei der Wellenlänge von 645 nm, weiterhin auf der korrigierten Näherungskurve $L_k$ liegt, das heißt, der Messwert E4 wird als Ankerpunkt für die Näherungskurve verwendet.

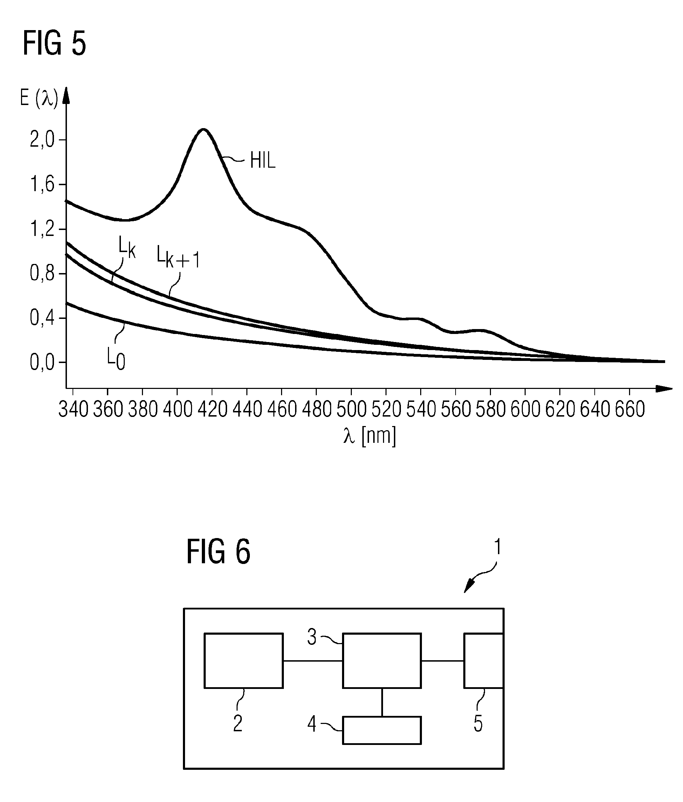

Fig. 5 zeigt eine schematische Darstellung des Diagramms aus Fig. 4, in welchem zusätzlich zu der ersten Näherungskurve $L_0$ eine korrigierte Näherungskurve $L_k$ dargestellt ist. Die korrigierte Näherungskurve $L_k$ hat insbesondere im blauen bzw. ultravioletten Wellenlängenbereich einen steileren Verlauf als die erste Näherungskurve $L_0$ und ist somit besser geeignet, den tatsächlichen Extinktionsanteil der in der Probe vorhandenen Lipide abzubilden.

In Schritt 33b können dann analog zu den Berechnungen in Schritt 33a die jeweiligen Extinktionsanteile $E_{L1}$, $E_{L2}$, $E_{L3}$ und $E_{L4}$ (= E4), der Lipide auf der Basis der korrigierten Näherungskurve $L_k$ korrigiert werden. Das Verfahren kann so lange mit den Schritten 34, 35, 36, 37, 38 und 33b iteriert werden, bis in Schritt 37 bestimmt wird, dass die Abweichung einen vorbestimmten Schwellwert unterschreitet. In diesem Fall können in Schritt 39 die korrigierten Näherungswerte für die Konzentrationen der Substanzen in der Körperflüssigkeitsprobe ausgegeben werden.

In Fig. 5 ist hierzu beispielhaft eine in einem nachfolgenden Iterationsschritt korrigierte Näherungskurve $L_{k+1}$ dargestellt, welche gegenüber der korrigierten Näherungskurve $L_k$ eine bessere Annäherung an den tatsächlichen Extinktionsanteil der in der Probe vorhandenen Lipide darstellt.

Fig. 6 zeigt eine schematische Darstellung eines Systems 1 zum Bestimmen der Konzentrationen von Substanzen in einer Körperflüssigkeitsprobe, insbesondere zum Durchführen des in Fig. 3 gezeigten Verfahrens 30. Das System 1 umfasst eine Messeinrichtung 2, eine Berechnungseinrichtung 3, einen Speicher 4 und eine Ausgabeeinrichtung 5.

Die Messeinrichtung 2 kann dazu ausgelegt sein, eine erste und zweite Substanzen enthaltende Körperflüssigkeitsprobe mit einem Lichtstrahl bei einer Vielzahl von Lichtwellenlängen zu durchstrahlen, und eine Vielzahl von Messwerten der Extinktion der Körperflüssigkeitsprobe bei der Vielzahl von Wellenlängen zu erfassen. Dazu kann die Messeinrichtung 2 beispielsweise Leucht- oder Laserdioden und entsprechende photometrische Sensoreinrichtungen aufweisen.

Die Berechnungseinrichtung 3 kann dazu ausgelegt sein, die Schritte 32, 33a, 34, 35, 36, 37, 38, 33b und 39 des Verfahrens 30 in Fig. 3 durchzuführen. Insbesondere können die ermittelten Näherungswerte für die Konzentrationen der Substanzen über die Ausgabeeinrichtung 5 an einen Nutzer des Systems 1 ausgegeben werden.

Der Speicher 4 kann dazu ausgelegt sein, vorbestimmte Werte für den Schwellwert und/oder Extinktionskoeffizienten zu speichern, die die Berechungseinrichtung 2 bei Bedarf abrufen kann.

**Patentansprüche**

1. Verfahren zum Bestimmen der Konzentrationen von mindestens zwei Substanzen (H; L) in einer Körperflüssigkeitsprobe, mit den Schritten:

   a) Durchstrahlen (31) der Körperflüssigkeitsprobe mit Licht bei einer Vielzahl von Wellenlängen und
   b) Erfassen einer Vielzahl von Messwerte der Extinktion der Körperflüssigkeitsprobe bei der Vielzahl von Wellenlängen;

   **gekennzeichnet durch** die Schritte:

   c) Erfassen eines ersten Messwertes (E4) bei einer ersten Wellenlänge und Bestimmen der Konzentration ($C_L$) der ersten Substanz (L) **durch** Division des Messwerts (E4) mit dem für die erste Substanz spezifischen Extinktionskoeffizienten;
   d) Berechnen (32) einer Näherungskurve ($L_0$) für die Extinktion der ersten Substanz (L) auf der Basis des ersten Messwerts (E4);
   e) Bestimmen (34) eines ersten Näherungswerts ($C_H$) der Konzentration der zweiten Substanz (H) auf der Basis eines zweiten Messwerts (E2) und Werten der Näherungskurve ($L_0$) bei einer zweiten Wellenlänge;
   f) Berechnen eines Extinktionswerts ($E_{HIL}$) bei einer dritten Wellenlänge auf der Basis des ersten Näherungswerts ($C_H$) und Werten der Näherungskurve ($L_0$) ;
   g) Ermitteln (36) einer Abweichung ($\Delta E$) des berechneten Extinktionswerts ($E_{HIL}$) von einem dritten Messwert (E1) bei der dritten Wellenlänge;
   h) Korrigieren (38) der Näherungskurve ($L_k$) auf der Basis der ermittelten Abweichung ($\Delta E$) ; und
   i) Bestimmen der Konzentration ($C_H$) der zweiten Substanz (H) **durch** Korrigieren des ersten Näherungswerts ($C_H$) auf der Basis des zweiten Messwerts (E2) und Werten der korrigierten Näherungskurve ($L_k$) .

2. Verfahren nach Anspruch 1, wobei ferner eine dritte Substanz (I) in der Körperflüssigkeitsprobe bestimmt wird, ferner mit den Schritten:

   j) Bestimmen (35) eines zweiten Näherungswerts ($C_I$) der Konzentration der dritten Substanz (I) auf der Basis des zweiten Messwerts (E2) und Werten der Näherungskurve ($L_0$) bei der zweiten Wellenlänge sowie eines vierten Messwerts (E3) und Werten der Näherungskurve ($L_0$) bei einer vierten Wellenlänge; und
   k) Bestimmen der Konzentration ($C_I$) der dritten Substanz (I) durch Korrigieren des zweiten Näherungswerts ($C_I$) auf der Basis des zweiten Messwerts (E2), des vierten Messwerts (E3) und Werten der korrigierten Nähe-

rungskurve ($L_k$),

wobei der Extinktionswert ($E_{HIL}$) zusätzlich auf der Basis des zweiten Näherungswertes ($C_I$) berechnet wird.

3. Verfahren nach Anspruch 2, wobei die Schritte des Berechnens des Extinktionswerts, des Ermittelns der Abweichung und des Korrigierens der Näherungskurve, des ersten Näherungswerts und des zweiten Näherungswerts so lange iteriert werden, bis die Abweichung ($\Delta E$) unterhalb eines vorbestimmten Schwellwerts liegt.

4. Verfahren nach einem der Ansprüche 2 bis 3, wobei die Körperflüssigkeitsprobe Blutserum oder Blutplasma umfasst, und wobei die erste Substanz (L) Lipide umfasst.

5. Verfahren nach Anspruch 4, wobei die zweite Substanz (H) Hämoglobin und die dritte Substanz (I) Bilirubin umfasst.

6. Verfahren nach einem der Ansprüche 2 bis 5, wobei die erste Wellenlänge im Bereich zwischen 610 nm und 650 nm, die zweite Wellenlänge im Bereich zwischen 410 nm und 420 nm, die dritte Wellenlänge im Bereich zwischen 360 nm und 370 nm und die vierte Wellenlänge im Bereich zwischen 465 nm und 475 nm liegt.

7. Verfahren nach Anspruch 3, wobei der vorbestimmte Schwellwert 10 mE beträgt.

8. Verfahren nach einem der Ansprüche 1 bis 7, wobei das Korrigieren der Näherungskurve derart erfolgt, dass der erste Messwert (E4) auf der Näherungskurve ($L_0$; $L_k$) liegt.

9. Verfahren nach einem der Ansprüche 1 bis 8, wobei das Durchstrahlen der Körperflüssigkeitsprobe mithilfe von Laser- oder Leuchtdioden und das Erfassen der Vielzahl von Messwerten (E1; E2; E3; E4) mithilfe eines photometrischen Sensors erfolgt.

10. System (1) zum Bestimmen der Konzentrationen von Substanzen in einer Körperflüssigkeitsprobe, mit:

    i. einer Messeinrichtung (2), welche dazu ausgelegt ist, eine Körperflüssigkeitsprobe mit Lichtstrahlen mit einer Vielzahl von Wellenlängen zu durchstrahlen, und eine Vielzahl von Messwerten der Extinktion der Körperflüssigkeitsprobe bei der Vielzahl von Wellenlängen zu erfassen; und
    ii. einer Berechnungseinrichtung (3), welche dazu ausgelegt ist, eine Näherungskurve für die Extinktion einer ersten Substanz (L) auf der Basis eines ersten Messwerts bei einer ersten Wellenlänge zu berechnen, einen ersten Näherungswert der Konzentration einer zweiten Substanz (H) auf der Basis eines zweiten Messwerts und Werten der Näherungskurve bei einer zweiten Wellenlänge zu bestimmen, einen Extinktionswert bei einer dritten Wellenlänge auf der Basis des ersten Näherungswerts und Werten der Näherungskurve zu berechnen, eine Abweichung des berechneten Extinktionswerts von einem dritten Messwert bei der dritten Wellenlänge zu ermitteln, die Näherungskurve auf der Basis der ermittelten Abweichung zu korrigieren und den ersten Näherungswert auf der Basis des zweiten Messwerts und Werten der korrigierten Näherungskurve zu korrigieren.

11. System (1) nach Anspruch 10, wobei die Messeinrichtung (2) Laser- oder Leuchtdioden und eine photometrische Sensoreinrichtung aufweist.

12. System (1) nach einem der Ansprüche 10 und 11, wobei die Berechnungseinrichtung (3) weiterhin dazu ausgelegt ist, einen zweiten Näherungswert der Konzentration einer dritten Substanz (I) auf der Basis des zweiten Messwerts und Werten der Näherungskurve bei einer zweiten Wellenlänge sowie eines vierten Messwerts und Werten der Näherungskurve bei einer vierten Wellenlänge zu bestimmen, und den zweiten Näherungswert auf der Basis des zweiten Messwerts und Werten der korrigierten Näherungskurve zu korrigieren, wobei der Extinktionswert zusätzlich auf der Basis des zweiten Näherungswertes berechnet wird.

13. System (1) nach Anspruch 12, wobei die Körperflüssigkeitsprobe Blutserum oder Blutplasma, die erste Substanz (L) Lipide, die zweite Substanz (H) Hämoglobin und die dritte Substanz (I) Bilirubin umfasst.

14. System (1) nach einem der Ansprüche 12 und 13, wobei die erste Wellenlänge im Bereich zwischen 610 nm und 650 nm, die zweite Wellenlänge im Bereich zwischen 410 nm und 420 nm, die dritte Wellenlänge im Bereich zwischen 360 nm und 370 nm und die vierte Wellenlänge im Bereich zwischen 465 nm und 475 nm liegt.

# FIG 1

# FIG 2

FIG 3

FIG 4

FIG 5

FIG 6

**Europäisches Patentamt**
**European Patent Office**
**Office européen des brevets**

# EUROPÄISCHER RECHERCHENBERICHT

Nummer der Anmeldung

EP 11 17 4320

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EP 1 059 522 A1 (HOFFMANN LA ROCHE [CH]) 13. Dezember 2000 (2000-12-13) * Zusammenfassung; Ansprüche 1,2 * ----- | 1-14 | INV. G01N21/27 |
| X | WO 2006/040387 A1 (THERMO ELECTRON OY [FI]; JOHANSSON HENRIK [FI]) 20. April 2006 (2006-04-20) S 9, Z 25 ff; * Zusammenfassung * ----- | 1-9 | |
| X | US 2010/174491 A1 (KIM HAN SANG [KR] ET AL) 8. Juli 2010 (2010-07-08) * Zusammenfassung; Abbildungen 1-2 * ----- | 1-14 | |
| X | WO 03/068060 A1 (DATEX OHMEDA INC [US]; HUIKU MATTI [FI]) 21. August 2003 (2003-08-21) * Ansprüche 1,24 * ----- | 1-14 | |

RECHERCHIERTE SACHGEBIETE (IPC)

G01N

Der vorliegende Recherchenbericht wurde für alle Patentansprüche erstellt

| Recherchenort | Abschlußdatum der Recherche | Prüfer |
|---|---|---|
| München | 28. November 2011 | Bigot-Maucher, Cora |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

........................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**                EP 11 17 4320

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

28-11-2011

| Im Recherchenbericht angeführtes Patentdokument | Datum der Veröffentlichung | Mitglied(er) der Patentfamilie | Datum der Veröffentlichung |
|---|---|---|---|
| EP 1059522 A1 | 13-12-2000 | EP 1059522 A1<br>EP 1185852 A1<br>JP 2003502631 A<br>US 6882425 B1<br>WO 0077494 A1 | 13-12-2000<br>13-03-2002<br>21-01-2003<br>19-04-2005<br>21-12-2000 |
| WO 2006040387 A1 | 20-04-2006 | EP 1802959 A1<br>US 2009009750 A1<br>WO 2006040387 A1 | 04-07-2007<br>08-01-2009<br>20-04-2006 |
| US 2010174491 A1 | 08-07-2010 | KR 20100082212 A<br>US 2010174491 A1 | 16-07-2010<br>08-07-2010 |
| WO 03068060 A1 | 21-08-2003 | AU 2003202603 A1<br>EP 1474036 A1<br>US 2003176776 A1<br>US 2005250998 A1<br>US 2009076354 A1<br>WO 03068060 A1 | 04-09-2003<br>10-11-2004<br>18-09-2003<br>10-11-2005<br>19-03-2009<br>21-08-2003 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1059522 A1
- US 4263512 A
- US 20090009750 A1
- US 20100174491 A1